# EUROPEAN PATENT APPLICATION

(11) **EP 1 130 032 A1**
(43) Date of publication of application: **05.09.2001**
(21) Application number: 00104082.3
(22) Date of filing: 28.02.2000
(51) Int. Cl.: C07K 16/28, C12N 15/13, C12N 15/70, C12N 1/21, G01N 33/577, G01N 33/574, A61P 43/00

(54) **Single-chain antibodies recognizing the human vascular endothelial growth factor receptor-2 (VEGFR-2/KDR)**

(71) Applicant: Gesellschaft für Biotechnologische Forschung mbH (GBF), 38124 Braunschweig (DE)
(72) Inventor: Boeldicke, Thomas, 38124 Braunschweig (DE); Weich, Herbert, 38124 Braunschweig (DE); Tesar, Michael, 82362 Weilheim (DE); Yayon, Avner, IL-76834 Moshav Sitria 104 (IL)
(74) Representative: Boeters, Hans Dietrich, Dr.

(57) **Abstract**

The invention relates to specific single-chain antibodies (scFv's) recognizing the human vascular endothelial growth factor receptor-2 (VEGFR-2/KDR) which show no crossreactivity to the human VEGF-receptor 1 (VEGFR-1). The scFv's have clinical application as a marker for angiogenesis in cryosections of different human tissues and may be used as a tool for FACS analysis and cell sorting of vascular endothelial cells, progenitor cells and hematopoitic stem cells, which are positive for VEGFR-2 gene expression.

### Annex to the application documents - subsequently filed sequences listing

SEQUENCE LISTING

<110> Gesellschaft f. Biotechnol. Forschung mbH (GBF)

<120> Single-chain antibodies recognizing the human vascular
      endothelial growth factor receptor-2 (VEGFR-2/KDR)

<130> 00104082.3

<140> EP 00104082
<141> 2000-02-28

<160> 4

<170> PatentIn Ver. 2.1

<210> 1
<211> 110
<212> PRT
<213> Artificial Sequence

<220>
<223> Description of Artificial Sequence: VH domain of a
      single-chain antibody recognizing the human
      vascular endothelial growth factor receptor-2

<400> 1
Gln Val Gln Leu Gln Gln Ser Gly Pro Glu Leu Val Lys Pro Gly Ala
  1               5                  10                  15

Ser Val Lys Ile Ser Cys Lys Thr Ser Gly Tyr Thr Phe Thr Glu Tyr
             20                  25                  30

Thr Met His Trp Val Lys Gln Ser His Gly Lys Ser Leu Glu Trp Ile
         35                  40                  45

Gly Gly Ile Asn Pro Asn Thr Gly Gly Thr Ser Tyr Asn Gln Lys Phe
     50                  55                  60

Lys Gly Lys Ala Thr Leu Thr Val Asp Lys Ser Ser Ser Thr Ala Tyr
 65                  70                  75                  80

Met Glu Leu Arg Ser Leu Thr Ser Glu Asp Ser Ala Val Tyr Tyr Cys
                 85                  90                  95

Ala Arg Gly His Tyr Ser Ser Ser Leu Asp Tyr Trp Gly Gln
            100                 105                 110

<210> 2
<211> 109
<212> PRT
<213> Artificial Sequence

<220>
<223> Description of Artificial Sequence: VH domain of a
      single-chain antibody recognizing the human
      vascular endothelial growth factor receptor-2

<400> 2
Gln Val Lys Leu Gln Glu Ser Gly Pro Glu Leu Val Lys Pro Gly Thr
  1               5                  10                  15

Ser Val Lys Ile Ser Cys Lys Thr Ser Gly Tyr Thr Phe Thr Glu Tyr
             20                  25                  30

Thr Met His Trp Val Lys Gln Ser His Gly Lys Ser Leu Glu Trp Ile
         35                  40                  45

Gly Gly Ile Asn Pro Asn Ser Gly Gly Thr Ser Tyr Asn Gln Lys Phe
     50                  55                  60

Lys Gly Lys Ala Thr Leu Thr Val Asp Lys Ser Ser Ser Thr Ala Tyr
 65                  70                  75                  80

Met Glu Leu Arg Ser Leu Thr Ser Glu Asp Ser Ala Val Tyr Tyr Cys
                 85                  90                  95

Ala Arg Gly Leu Trp Gly Gly Met Asp Tyr Trp Gly Gln
            100                 105

<210> 3
<211> 99
<212> PRT
<213> Artificial Sequence

<220>
<223> Description of Artificial Sequence: VL domain of a
      single-chain antibody recognizing the human
      vascular endothelial growth factor receptor-2

<400> 3
Thr Ile Met Ser Ala Ser Pro Gly Glu Lys Val Thr Met Thr Cys Ser
  1               5                  10                  15

Ala Ser Ser Ser Val Ser Tyr Met His Trp Tyr Gln Gln Lys Ser Gly
            20                  25                  30

Thr Ser Pro Lys Arg Trp Ile Tyr Asp Thr Ser Lys Leu Ala Ser Gly
        35                  40                  45

Val Pro Ala Arg Phe Ser Gly Ser Gly Ser Gly Thr Ser Tyr Ser Leu
    50                  55                  60

Thr Ile Ser Ser Met Glu Ala Glu Asp Ala Ala Thr Tyr Tyr Cys Gln
65                  70                  75                  80

Gln Trp Thr Ser Asn Pro Phe Thr Phe Gly Ser Gly Thr Lys Leu Glu
                85                  90                  95

Ile Lys Arg

<210> 4
<211> 99
<212> PRT
<213> Artificial Sequence

<220>
<223> Description of Artificial Sequence: VL domain of a
      single-chain antibody recognizing the human
      vascular endothelial growth factor receptor-2

<400> 4
Ala Ile Met Ser Ala Ser Pro Gly Glu Lys Val Thr Leu Thr Cys Ser
 1               5                  10                  15

Ala Ser Ser Ser Val Ser Tyr Met His Trp Tyr Gln Gln Lys Ser Ser
            20                  25                  30

Thr Ser Pro Lys Leu Trp Ile Tyr Asp Thr Ser Arg Leu Ala Ser Gly
        35                  40                  45

Val Pro Gly Arg Phe Ser Gly Ser Gly Ser Gly Thr Ser Tyr Ser Leu
    50                  55                  60

Thr Ile Ser Ser Met Glu Ala Glu Asp Ala Ala Thr Tyr Tyr Cys Gln
65                  70                  75                  80

Gln Trp Asn Thr Tyr Pro Tyr Thr Phe Gly Gly Gly Thr Lys Leu Glu
                85                  90                  95

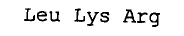

## Description

The invention relates to single-chain antibodies recognizing the human vascular endothelial growth factor receptor-2 (VEGFR-2/KDR) on the surface of primary endothelial cells and preselected CD34⁺ cells from cord blood.

The following abbreviations are used througout this specification: FACS, fluorescence-activated cell scanner; FITC, fluorescein isothiocyanate; HRP, horseradish peroxidase; HUVE cell, human umbilical vein endothelial cell; KDR, kinase insert domain-containing receptor; OPD = 1,2-phenylenediamine-dihydrochloride; PAE cells, Porcine aortic endothelial cells; PE, phycoerythrin; scFv, single-chain fragment of the variable domain; TRITC, tetramethylrhodamine; VEGF, vascular endothelial growth factor; VEGFR-1, -2, vascular endothelial growth factor receptor-1, -2.

A reference list with more detailed bibliographic information can be found at the end of this specification.

### Introduction and Background

Angiogenesis, the formation of new blood vessels, is crucial both during embryonic development and in the maintenance of normal physiological processes combined with the development of new blood vessels like wound repair, tissue regeneration and during the menstrual cycle (Ferrara and Smyth, 97). There are a variety of pathophysiological disorders like retinopathies, rheumatrid arthritis and tumor growth, in which angiogenesis is a prerequisite for the progression of the disease. Vascular endothelial growth factor (VEGF or VEGF-A) is a key growth factor and vascular permeability factor for endothelial cells (Ferrara et al., 1996). VEGF-A is not only a specific growth factor for vascular endothelial cells but also a potent inducer and prime regulator of blood vessel formation for in vivo experiments or in physiologically occurring new vessel formation (Leung et al., 1989; Wilting et al., 92). Very recently, several new members of the VEGF growth factor family have been described and were consequently named VEGF-B to VEGF-E. All these growth factors together with placenta growth factor (PlGF) mediate their biological activity and signals through three receptor tyrosine kinases called VEGFR-1 (flt-1), VEGFR-2 (KDR/flk-1) and VEGFR-3 (flt-4) (Veikolla and Alitalo, 1999.) From the three receptors, VEGFR-2 is expressed exclusively on vascular endothelial cells and on hematopoietic cells (Millauer et al., 1993; Quinn et al., 1993; Asahara et al., 1997; Ziegler et al., 1999). During formation of new blood vessel or in tumor angiogenesis, the VEGFR is upregulated but down regulation in normal vessels (Risau, 1997). An exception is the human kidney where upregulation of VEGFFR-2 gene expression is connected with vascular permeability in the kidney glomerulus (Simon et al., 1998).

Previously, the production of a rat anti-VEGFR-2 monoclonal antibody (Rockwell et al., 1995) with neutralizing activity has been reported together with several other monoclonal antibodies against the human VEGFR-2 suitable for Western blotting and ELISA (Menrad et al., 1997). More recently, several single-chain antibodies against VEGFR-2 has been generated which were able to block binding of VEGF-A to its receptor (Zhu et al., 1998). These antibodies were able to block VEGF-A induced DNA-Synthesis and phosphorylation of the receptors. Very recently and for the first time it was demonstrated, that pluripotent hematopoietic stem cells (HSCs) can be purified from bone marrow and cord blood by using a combination of CD34+ antibodies followed by VEGFR-2 antibodies for cell sorting of native cells (Ziegler et al., 1999). The VEGFR-2 antibody used in this study is a high affinity antibody, which has been described from the inventors working group before (Roeckl et al., 1998) generated against the soluble, extracellular domain of VEGFR-2.

An object of the invention is to establish a novel generation of single-chain antibodies for a broad spectrum of analytical assays and their employment as high affinity antibodies for the isolation and characterization of primary human endothelial cell and other progenitor cell types, known to be positive for VEGFR-2 gene expression.

These and other objects and features of the invention will be apparent from the description, drawings, and claims which follow.

Accordingly, the invention provides a single-chain antibody as defined in claim 1 recognizing the human vascular endothelial growth factor receptor-2 selected from the group consisting of:
(a) a polypeptide having the following general structure:
   (V_{H} domain)-Linker-(V₁ domain),
   wherein said V_{H} domain comprises the following amino acid sequence: 
Gln Val Gln Leu Gln Gln Ser Gly Pro Glu Leu Val Lys Pro Gly
Ala Ser Val Lys Ile Ser Cys Lys Thr Ser Gly Tyr Thr Phe Thr
Glu Tyr Thr Met His Trp Val Lys Gln Ser His Gly Lys Ser Leu
Glu Trp Ile Gly Gly Ile Asn Pro Asn Thr Gly Gly Thr Ser Tyr
Asn Gln Lys Phe Lys Gly Lys Ala Thr Leu Thr Val Asp Lys Ser
Ser Ser Thr Ala Tyr Met Glu Leu Arg Ser Leu Thr Ser Glu Asp
Ser Ala Val Tyr Tyr Cys Ala Arg Gly His Tyr Ser Ser Ser Leu
Asp Tyr Trp Gly Gln- (scFv A2)
 or 
Gln Val Lys Leu Gln Glu Ser Gly Pro Glu Leu Val Lys Pro Gly
Thr Ser Val Lys Ile Ser Cys Lys Thr Ser Gly Tyr Thr Phe Thr
Glu Tyr Thr Met His Trp Val Lys Gln Ser His Gly Lys Ser Leu
Glu Trp Ile Gly Gly Ile Asn Pro Asn Ser Gly Gly Thr Ser Tyr
Asn Gln Lys Phe Lys Gly Lys Ala Thr Leu Thr Val Asp Lys Ser
Ser Ser Thr Ala Tyr Met Glu Leu Arg Ser Leu Thr Ser Glu Asp
Ser Ala Val Tyr Tyr Cys Ala Arg Gly Leu Trp Gly Gly Met Asp
Tyr Trp Gly Gln- (scFv A7),
 and wherein said V_{L} domain comprises the following amino acid sequence: 
-Thr Ile Met Ser Ala Ser Pro Gly Glu Lys Val Thr Met Thr Cys
Ser Ala Ser Ser Ser Val Ser Tyr Met His Trp Tyr Gln Gln Lys
Ser Gly Thr Ser Pro Lys Arg Trp Ile Tyr Asp Thr Ser Lys Leu
Ala Ser Gly Val Pro Ala Arg Phe Ser Gly Ser Gly Ser Gly Thr
Ser Tyr Ser Leu Thr Ile Ser Ser Met Glu Ala Glu Asp Ala Ala
Thr Tyr Tyr Cys Gln Gln Trp Thr Ser Asn Pro Phe Thr Phe Gly
Ser Gly Thr Lys Leu Glu Ile Lys Arg (scFv A2)
 or 
-Ala Ile Met Ser Ala Ser Pro Gly Glu Lys Val Thr Leu Thr Cys
Ser Ala Ser Ser Ser Val Ser Tyr Met His Trp Tyr Gln Gln Lys
 
Ser Ser Thr Ser Pro Lys Leu Trp Ile Tyr Asp Thr Ser Arg Leu
Ala Ser Gly Val Pro Gly Arg Phe Ser Gly Ser Gly Ser Gly Thr
Ser Tyr Ser Leu Thr Ile Ser Ser Met Glu Ala Glu Asp Ala Ala
Thr Tyr Tyr Cys Gln Gln Trp Asn Thr Tyr Pro Tyr Thr Phe Gly
Gly Gly Thr Lys Leu Glu Leu Lys Arg (scFv A7);
(b) a polypeptide which is an allelic variant of a polypeptide defined in (a) and has the same antibody activity;
(c) a mutant of a polypeptide defined in (a) or (b) in which one or more amino acid residues are deleted and/or replaced and/or inserted and/or one or more partial amino acid sequences are inverted without interfering with the antibody activity or which enhance the antibody activity;
(d) a C-terminal or N-terminal fusion protein comprising a polypeptide defined in (a) to (c) in which the additively connected amino acid residues do not interfere with the antibody activity or may be easily eliminated.

Further, the invention provides a DNA-Sequence as defined in claim 7 comprising a DNA sequence selected from the group consisting of:
(a) a DNA sequence coding for a single-chain antibody according to any of claims 1 to 3, or its complementary strand;
(b) a DNA sequence which hybridizes under stringent conditions to the polypeptide coding regions of a DNA sequence defined in (a), or fragments thereof;
(c) a DNA sequence which, but for the degeneracy of the genetic code, would hybridize to a DNA sequence defined in (a) and (b);
(d) allelic Variations of a DNA sequence defined in (a) and resulting in isofunctional expression products;
(e) mutants of a DNA sequence defined in (a) to (d) in which one or more nucleotide residues are deleted and/or replaced and/or inserted and/or one or more partial nucleotide sequences are inverted and resulting in isofunctional expression products.

In addition, the invention provides a recombinant expression vector as defined in claim 8, a procaryotic or eucaryotic cell as defined in claim 9, a process for the preparation of a single-chain antibody of the invention as defined in claim 11, and an expression product or partial expression product as defined in claim 12.

Further, the invention provides the use of the single-chain antibody as defined above as an immunohistochemical marker for angiogenesis or vascularization in a human tissue, for FACS analysis and cell sorting of cells expressing human vascular endothelial growth factor receptor-2 on their surfaces, and for vascular and stem cell targeting a drug or toxin, a radionuclide, a gene or a viral coat protein conjugated to said single-chain antibody.

Moreover, the invention provides a kit comprising at least one single-chain antibody as defined above for the uses defined above.

Further advantageous and/or preferred embodiments of the invention are subject-matter of the subclaims.

Thus, in one aspect the invention relates to a single-chain antibody as defined above, wherein said linker is a flexible peptide comprising 2 to 28 amino acid residues, and is for example (Gly₄Ser)₃ or the known Yol-peptide linker.

In another aspect the invention relates to a single-chain antibody as defined above, wherein said single-chain antibody is labeled without interfering with the antibody activity, for example with a label selected from the group consisting of one or more radioactive atoms or groups, coloured or fluorescent groups, haptens, enzymes, magnetic particles, groups for immobilization to solid phases and groups for direct or indirect reactions, wherein said groups for indirect reactions are for example based on or are suitable for enzyme-conjugated secondary antibodies, the biotin/avidin(or streptavidin) system or the colloidal gold system.

The abovementioned transformed or transfected cell for example may be derived from an E. coli strain.

Recombinant single chain antibodies as described in this invention have the following advantages compared to monoclonal antibodies derived from hybridomas:
They could more easily expressed and prepared in bacteria; they could be easily purified from the bacterial periplasm or supernatant; no risk of contamination with serum-components (for example virus contamination); no expensive cell-culture medium and expensive cell-culture laboratory; ecomomical, cheap generation and purification of the recombinant antibodies; because of the small size (1/6 smaller than monoclonal antibodies derived from hybridomas) they penetrate better into cells and tissue, therefore they can be better used for in vivo imaging and in vivo diagnostic; single-chain antibodies could easily be fused to a drug, toxin, radionuclide, a gene or a viral coat protein.

The single-chain antibody as defined above for example may be used as an immunohistochemical marker for angiogenesis or vascularisation in cryosections from human tissue, for example for imaging a growing carcinoma.

Further, the single-chain antibody as defined above may for example be used for binding to, targeting, selection and isolation of cells expressing human vascular endothelial growth factor receptor-2, for example selected from the group consisting of human vascular endothelial cells and their progenitor cells, human megacaryocytes and their progenitor cells, human hematopoitic stem cells from bone marrow, umbical cord blood or mobilized peripheral blood.

Cell sorting may for example be performed by using flow cytometry or by applying antibody or magnetic particle mediated selection and without blocking growth factor induced cell proliferation and differentiation during selection and propagation.

### Brief description of the drawings

The foregoing and other objects of the invention, the various features thereof, as well as the invention itself, may be more fully understood from the following description, when read together with the accompanying drawings, in which:

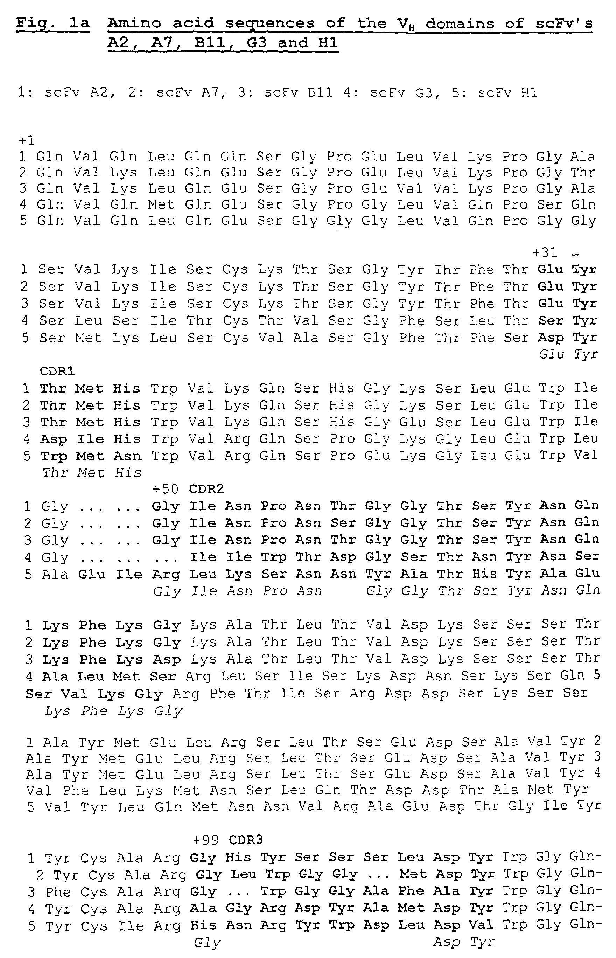

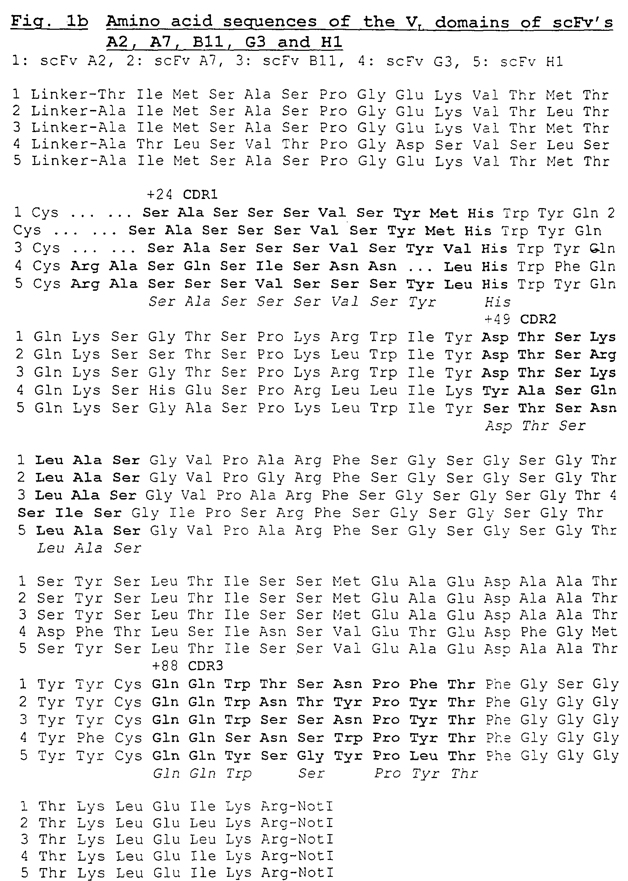

Fig. 1 shows the deduced amino acid sequences of the V_{H} and V_{L} domains of the scFv fragments A2, A7, B11, G3 and H1 isolated from the phage display library:
   (A) Amino acid sequences of the V_{H} domains . The complementary-determing regions (CDR1-CDR3) are printed in bold according to Kabat et al., 1991. Sequences were aligned using the Multalin program. Consensus sequences are printed in italics. (B) Amino acid sequences of the V_{L} domains. Linker: Amino acid sequence (Gly₄Ser)₃ between the V_{H} and V_{L} domains; NotI: restriction site used for ligation of the assembled scFv into the pCANTAB 5E vector.

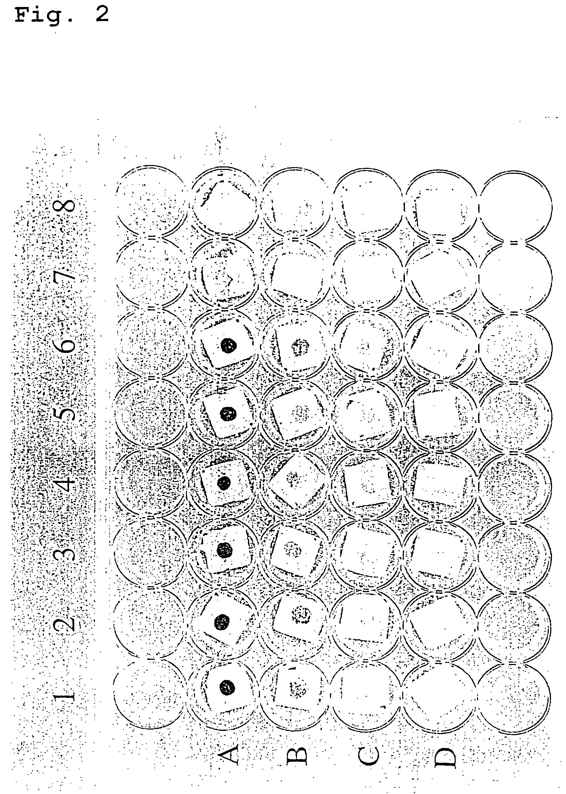

Fig.2 shows an immunoblot analysis: Binding of the anti-VEGFR2 scFv fragments to native and denatured VEGFR-2:
   The antigen (0.1 µg/2.5µl) was spotted onto several scraps of a cellulose membran which has been placed into the wells of a 48-well microtitre-plate. Treatments of the VEGFR-2 were as follows: (A) native; (B) denaturation with PBS containing 2% SDS for 10 min at 37°C, (C) denaturation with the same buffer as in (B) for 5 min at 95°C; (D) denaturation with PBS containing 2% SDS and 1% DTT (Laemmli-buffer). After the blocking step the scraps were treated with the scFv-fragments (1) A2, (2) A7, (3) B11, (4) G3, (5) H1 and (6) polyclonal mouse serum (diluted 1: 500 in PBS). Column (7) and (8) are background controls for the secondary antibodies HRP-labelled anti-E tag antibody without scFv-fragment (7) and HRP labelled goat anti-mouse IgGFc antibody without polyclonal mouse serum (8) respectively.

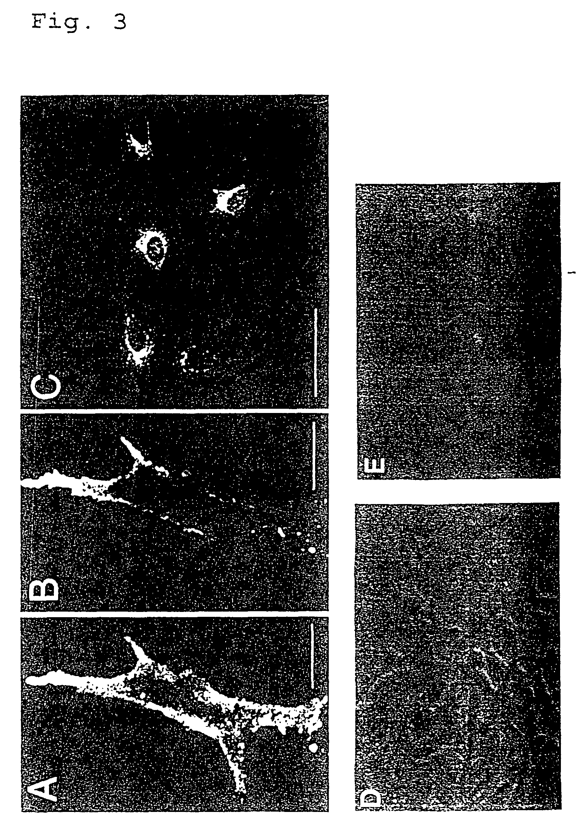

Fig. 3 shows the immunofluorescence of scFv A7 with adhärent PAE/VEGFR-2 cells analyzed by laser scanning confocal microscopy:
   Confocal Imaging, three sections of a Z-Series are depicted:
   (A) Confocal section on the surface area of the cell. (B) Section 2 µm underneath the surface area of the cell. (C) Cells fixed with aceton/methanol, section 4 µm underneath the surface area of the cell. The bars represents in (A) and (B) 10 µm and in (C) 50 µm. (D) and (E) are negative controls with PAE/VEGFR-1 cells: (D) Light microscopy, (E) Immunofluorescence; in (D) and (E) cells were analysed with a photo-microscope (Zeiss Axiophot), magnification: x 175.
Fig.4 shows the laser-scanning confocal micrographs of PAE/VEGFR-2 cells in suspension incubated with scFv A7 (I) and scFv A2 (II):
   (I, A-D) A z-axis section series from top to bottom of a representative cell after incubation with scFv A7; section thickness is 2 µm. Bar represents 10 µm. (II, A-D) A z-axis section series as in (I, A-D), representative cell after incubation with scFv A2. Bar represents 5 µm.
Fig. 5 shows the immunohistology of frozen human kidney tissue sections with scFv A7:
   (A) Cryosection of a interlobular artery. A monolayer of cells inside the artery with a red positive label for VEGFR-2 is shown. (B) Negative control with the secondary antibodies: HRP-labelled mouse anti-E tag antibody, rabbit anti-mouse monoclonal antibody and alkaline-phosphatase labelled mouse antibody without scFv A7. (C) Glomerulus with a red positive label for VEGFR-2 in capillaries. (D) Negative control with the secondary antibodies as in (B). Nuclei are stained with hämatoxilin.

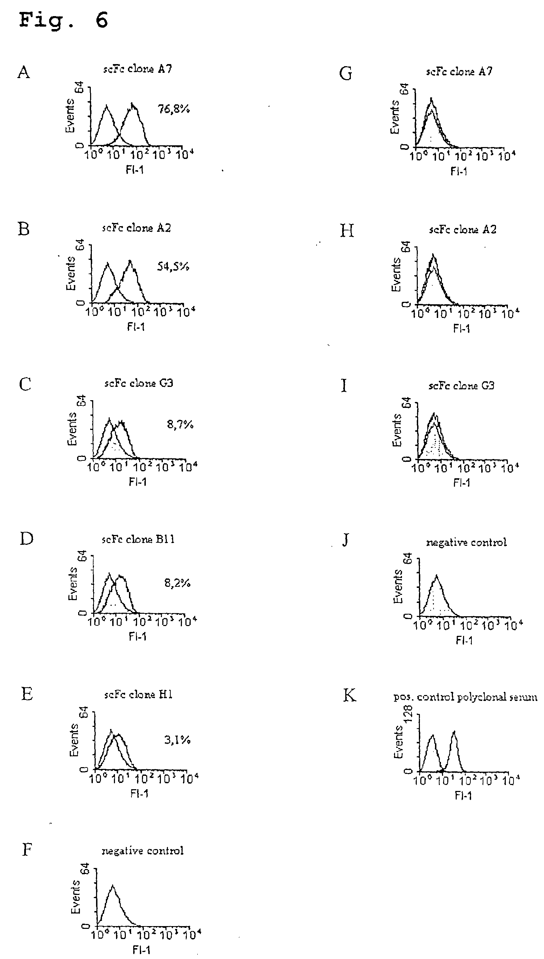

Fig. 6 shows the binding of anti-VEGFR-2 scFv fragments to PAE/VEGFR-2 cells analysed by flow cytometry:
   Shown is the binding of the scFv-fragments A7 (A), A2 (B), G3 (C), Bll(D) and H1(E) to PAE/VEGFR-2 cells. Percentages represent the number of gated living VEGFR-2 positive cells detected with the corresponding antibody. (F) shows the negative control with the secondary antibodies: HRP-labelled mouse anti-E tag antibody and FITC-conjugated sheep anti-mouse IgG antibody. Binding of the five scFv-fragments to PAE/VEGFR-1 cells was negative. Representatively are shown the clones A7 (G), A2 (H) and G3 (I). (J) shows the negative control with the secondary antibodies without the primary antibodies and (K) shows the positive control with a rabbit polyclonal anti-VEGFR-1 serum. In this case binding was detected with a FITC labelled goat anti-rabbit IgG (H+L) antibody.

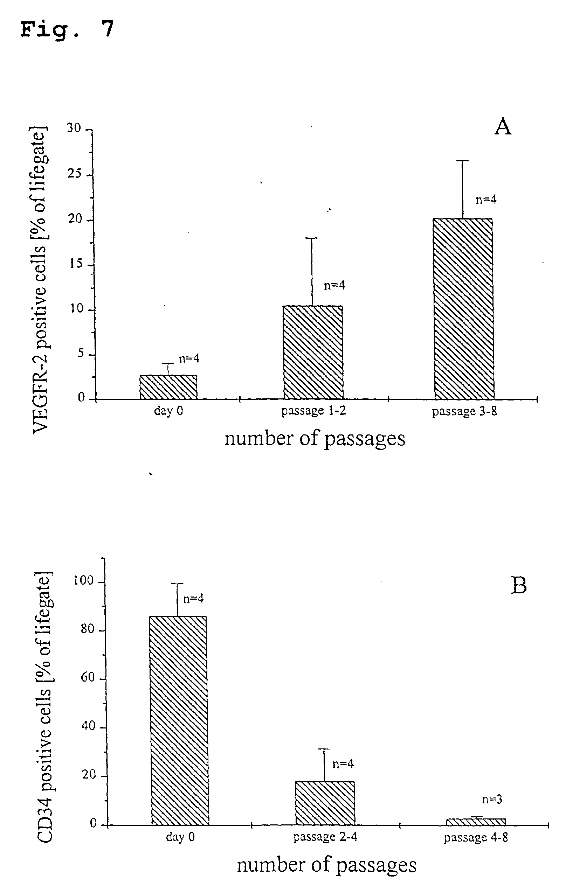

Fig.7 shows the amount of VEGFR-2- and CD34 -expressing HUVE cells in function of the number of cell passages analysed with scFv A7 by flow cytometry:
   (A) HUVE cells were freshly prepared from the umbivilical cord (day 0), cultivated over different passages and the percentage of gated living VEGFR-2 positive cells determined with scFv A7. Bars represents the mean deviation of 4 different experiments. (B) The amount of gated living CD34 positive cells was determined in parallel. Bars represent as in (A) the mean deviation of 4 and 3 different experiments respectively.

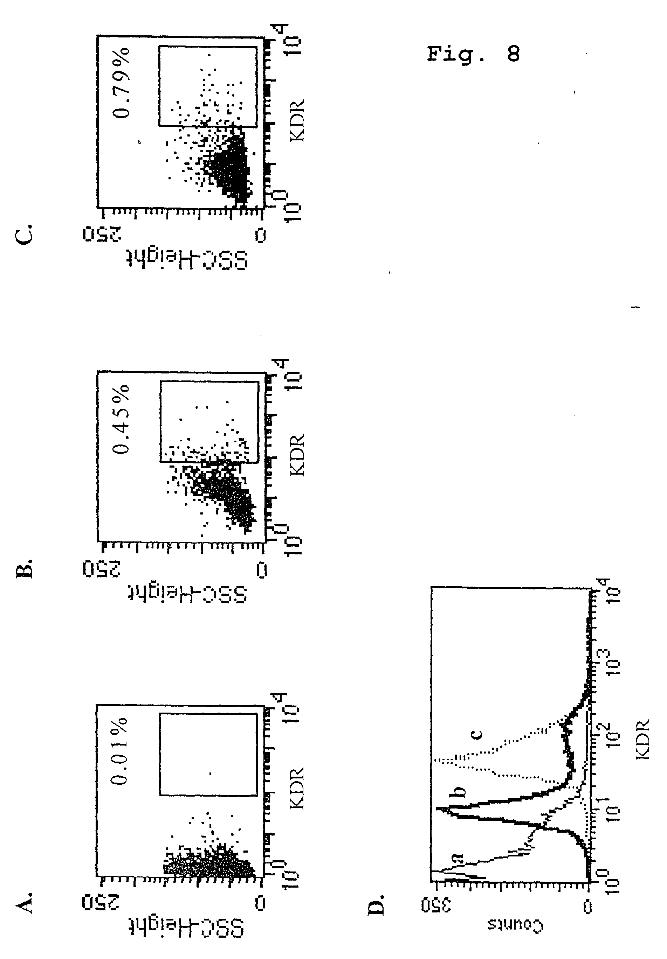

Fig.8 shows the flow cytometry analysis of VEGFR-2 expression on human cord blood CD34⁺ cells with scFv A7:
   Enriched human cord blood CD34⁺ cells were stained with streptavidine-PE (A), negative control, biotinilated anti-VEGFR-2 mAb clone 260.4 (B) or scFv A7 (C). Indicated are the amounts (%) of CD34⁺ cells expressing VEGFR-2. (D) shows staining of HUVE-cells with (a) non relevant Mouse IgG-PE, (b) biotinilated anti-VEGFR-2 mAb clone 260.4 and (c) scFv A7.

In the following the invention is disclosed in more detail with reference to examples and to drawings. However, the described specific forms or preferred embodiments are to be considered in all respects as illustrative and not restrictive, the scope of the invention being indicated by the appended claims rather than by the following description, and all changes which come within the meaning and range of equivalency of the claims are therefore intended to be embraced therein.

### Material and methods

### Cell Lines and Proteins

The soluble VEGFR-2 protein was expressed in insect cells and purified as described in detail before. It consists of the first seven Ig-like domains of the extracellular receptor part (Roeckl et al., 1998). Human VEGFR-1 and VEGFR-2 transfected porcine aortic endothelial cells (PAE/VEGFR-1/VEGFR-2 cells) were described earlier by Waltenberger et al., 1994 and cultivated in Ham's F12 medium containing 10% fetal calf serum (Seromed, Berlin, Germany). HUVE cells were prepared according to Quadros et al., 1989 and cultivated in endothelial cell Basal Medium-2 (Clonetics, San Diego, CA).

Human cord blood mononuclear cells from full termed deliveries were isolated by standard separation on Ficoll-Paque (Amersham Pharmacia Biotech). Enrichment of CD34+ cells was performed using a MACS separation kit (Miltenyi Biotech, Bergisch Gladbach, Germany). Adherent HUVE cells were removed for FACS staining using 10 mM EDTA. All procedures performed with the human cord blood mononuclear cells were performed at the Weizmann Instititut of Science and approved by the human experimentation and ethics committees.

All cells were grown under normoxic conditions with 5% CO2 in a humidified cell culture incubator (Heraeus, Hanau, Germany). All chemicals, if not indicated otherwise, were obtained from Sigma-Aldrich Chemie GmbH (Deisenhofen, Germany).

### Construction of the Immune V-gene phage display library and selection of specific scFv-fragments by biopanning.

3 BALB/c mice (Zentralinstitut für Versuchstierzucht, Hannover, Germany) were injected into the feet pads, each mouse with 10 µg of the extracellular domän of VEGFR-2 in 50µl PBS (145 mM NACl, 7.5 mM Na₂HPO₄, 2.5 mM NaH₂PO₄x2H₂O, pH 7.1) mixed with 50µl complete Freund's adjuvant. Immunizations were repeated at days 7, 15, and 23 with incomplete adjuvant. At day 28 the poplitheal lymph nodes were removed and the B-cells isolated out of the tissue. After extraction of the total RNA according to Chomczynski and Sacchi (1987), mRNA was isolated using the mRNA isolation kit (Quiagen, Hilden, Germany). After first strand cDNA synthesis using the "First strand cDNA synthesis kit" from Amersham Pharmacia Biotech (Freiburg, Germany), the V_{H} and V_{L} domains were amplified using the primers published by Clackson et al., 1991 and McCafferty et al., 1996. All primers were synthesized by Gibco, BRL, Eggenstein, Germany). Amplification reactions were performed in 5 different PCR tubes (1 tube for amplification of the heavy chains using the VHlBack and the VH1FOR-2 primer; the other 4 tubes were used for amplification of the V_{K} light chains employing the V_{K} 2Back primer and one of the 4 J-region primers (MJK1FONX, MJK2FONX, MJK4FONX, MJK5FONX), McCafferty et al., 1996. After purification on an 1.5% agarose gel the amplified Heavy chain DNA was distributed to 4 different PCR tubes and the amplified Light chain DNA after reaction with the corresponding J-region primer added. The scFv fragments were assembled and restriction sites incorporated using the "Recombinant Phage Antibody System" (Amersham Pharmacia Biotech). The assembled scFv DNA was pooled, purified on an 1.5% agarose gel, and ligated into the pCANTAB 5E vector (Amersham Pharmacia Biotech) using *SfiI/NotI* restriction sites and transformed into *E.coli* XL-1-Blue cells by electroporation. Transformed XL-1-Blue cells were plated onto SOB-A-G-T plates (20 g Bacto-tryptone, 5g Bacto-yeast extract, 0.5 g NaCl per liter medium, plus 2% glucose, 100µg/ml ampicillin and 10µg/ml tetracyclin) and incubated overnight at 30°C. On the next day cells were scraped into 25 ml 2YT-G-A-T medium (17g Bacto-tryptone, 10g Bacto-yeast extract, 5g NaCl, plus 2% glucose, 100µg/ml ampicillin and 10µg/ml tetracyclin) and diluted until OD₆₀₀ of 0.4 was reached. Then cells were grown to OD₆₀₀ of 0.6 and phagemids rescued with M13K07 helper phage and amplified overnight in 2YT-A-T medium at 30°C. The phages were precipitated with 4% PEG/0.5 M NaCl and resuspended in lml PBS containing 0.02% Na-acid.

For Biopanning microtiterplates (MaxiSorb™ polystyrene assay plates, Nunc, Wiesbaden, Germany) coated with 10µg/ml of the extracellular domain of VEGFR-2 were blocked with 5% skimmed milk for 30 min at room temperature. Then 2.5 x 10⁹ colonie forming units (cfu) phagemid rescue in 100µl 5% skimmed milk (blocked with this solution for 30 min at room temperature) were incubated for 2h at room temperature. The wells were washed during the first panning 12 x with PBS and during the 2^{nd} and 3^{rd} panning 12 x with PBS containing 0.05% Tween 20 (PBST) followed by washing with 12 x PBS. Bound phages were eluted with 200 µl glycin-HCL (pH 2.2) by incubation for 15 min at room temperature. After neutralisation with a saturated Tris solution 800 µl eluted phages (from 4 wells) were incubated with 800 µl log-phase *E.coli* XL-1 Blue for lhr at 37°C, infected cells plated onto SOB-A-G-T plates and incubated overnight at 30°C.

### Phage ELISA

Individual clones obtained after the third panning were grown overnight at 30°C in 96-well microtiterplates in 2YT-G-A-T medium and rescued with helper phage as described above. Phage antibody containing supernatant was blocked by diluting 1:2 in 5% skimmed milk for lh at room temperature, added to 96-well microtiterplates (MaxiSorb™ polystyrene plates, Nunc), coated with 1µg/ml of the antigen and incubated for 2 h at room temperature. Plates were washed 10 x with PBST and incubated with a HRP labelled mouse anti-M13 antibody for lh at room temperature (Amersham Pharmacia Biotech). The plate was developed for 10-20 minutes with a OPD-solution (10 mg in 25 ml phosphate-citrate buffer), the reaction stopped by adding 10% H₂SO₄ and absorption measured at 490 nm. The DNA of positive clones was sequenced using the pCANTAB 5 sequencing primer set (Amersham Pharmacia Biotech) and analyzed with a ABI PRISM™ 310 Genetic Analyzer (Perkin-Elmer, Weiterstadt, Germany).

### Preparation of soluble scFv.

The nonsuppressor *E. coli* host HB2151 was infected with individual positive phages and infected cells selected on SOB-A-G plates at 30°C overnight. Next day cells were cultured in 2YT-A-G (0.1% glucose) medium to OD₆₀₀ of 1.0. Expression of scFv-fragments was induced with 1mM IPTG, cells grown overnight at 30°C and antibodies harvested from the supernatant after centrifugation. Expression of the scFv's was analyzed in an ELISA with the extracellular domain of VEGFR-2 as antigen using a HRP labelled mouse anti-E tag antibody (Pharmacia Amersham Biotech). Soluble antibodies were purified using a HiTrap anti-E Tag column from Amersham Pharmacia Biotech (RPAS Purification Module).

### Immunoblot analysis of the scFv's with native and denatured VEGFR-2 antigen.

The antigen was spotted onto a cellulose membran in its native, untreated state or denaturated. The membrane was blocked with 5% skimmed milk in PBS for lh and incubated with the purified scFv's (2µg/ml) for 2h. Bound antibodies were detected with the HRP labelled mouse anti-E tag antibody (diluted 1:2500 in 2% skimmed milk). The binding of a polyclonal serum obtained from one of the three immunized mice was detected with a HRP labelled goat-anti mouse IgG-Fc antibody (diluted 1:1000 in 2 % skimmed milk, Dianova, Hamburg, Germany). All incubation steps with the secondary antibodies were performed at room temperature for lh and in between times the membran was washed 5 times with washing buffer (PBS/0.05% Tween 20). Blots were developed with OPD (see Phage ELISA) for 5-10 min, washed 2 times with PBS and photographed.

### Cell Surface Immunofluorescence

Immunofluorescence with adhärent cells: PAE/VEGFR-2 cells were grown for 48 h on sterile coverslips coated with 0.1% gelatine in HAM's F-12 Medium containing 10% FCS (v/v) and 0.4mg/ml G418. After discarding the medium, cells were directly incubated for lh with 0.5 µg/ml (diluted in PBS/2% FCS) of purified antibody, followed by a 1h-incubation with the HRP labelled anti-E tag antibody (diluted 1 : 2500 in PBS/2%FCS) and a lh-incubation with a TRITC-labelled goat-anti-mouse IgG (H+L) antibody (diluted 1: 160 in PBS/2% FCS, Dianova). All incubation steps were performed at 4°C in the dark and inbetween these steps washing was performed 4 times with PBS/1% BSA. Then cells were fixed with 1% paraformaldehyde in PBS for 30 min on ice, washed 4 times, embedded in moviol (Hoechst, Germany) and analyzed the next day with the confocal microscope. For intracellular staining cells were fixed with aceton/methanol for 30 sec at room temperature.

Immunofluorescence with PAE/VEGFR-2 cells in suspension: adherent growing PAE/VEGFR-2 cells were detached by incubation with 5mM EDTA pH 7.1 at room temperature. The cells were centrifuged at 1000 rpm for 5 min at 4°C, washed with PBS/2%FCS and 5 x 10⁴ cells were incubated with 0.5 µg/ml scFv in 100 µl PBS/2% FCS in a 96-well microtiter plate for 1 h at 4°C. The following incubation steps with the secondary antibodies were the same as described above with the adherent cells. After each incubation step washing was performed one times with PBS/2% FCS. Cells were fixed with 1% paraformaldehyde, washed two times, resuspended in 50 µl PBS and fixed for 10 min at room temperature on polylysin coated coverslips, embedded in moviol and analysed the next day with the confocal microscope.

### Immunohistochemistry

5 µm frozen tissue sections were prepared from adult kidneys which were received immediately after surgical removel from patients suffering from renal carcinoma. Tissue sections, fixed in acetone at -10°C for 10 min were incubated with 140 µg/ml scFv A7 for 2h at 22°C. Then sections were incubated with the HRP-labelled mouse anti-E tag antibody (1:2500) at 22°C for 1 h, followed by a 1h incubation with a rabbit anti-mouse antibody (Z 259, DAKO) and a 1h incubation with alkaline-phosphatase labelled mouse monoclonal antibody (1: 40). All dilutions were performed in PBS pH 7.6. Bound antibodies were visualized with a solution of sodium nitrite (28 mM), new fuchsin (basic fuchsin, 21 mM), naphthol-AS-BI-phosphate (0.5 mM), dimethylformamide (64 mM), and levamisole (5mM) in 50 mM Tris/HCL buffer, pH 8.4, containing 146 mM NaCl (Incubation time: 15 min). Nuclei were stained with hämatoxilin. Washing was performed with H₂O and PBS.

### FACS-analysis

FACS analysis were performed with scFv A7 using (a) recombinant PAE/VEGFR-1 and PAE/VEGFR-2 cells, (b) HUVE cells and (c) CD34⁺ cells isolated from cord blood.
(a) adherent grown PAE/VEGFR-2 and PAE/VEGFR-1 cells were detached from the bottom of a flask by incubation with a cold Trypsin/EDTA solution (1x, Sigma). After washing cells with PBS/2% FCS they were directly used for FACS analysis. All incubation steps with antibodies were performed for 30 min at 4°C in a 96-well microtitre plate (Nunclon™ Surface plate, Nunc) as described in section immunofluorescence with cells in suspension. Binding of the scFv's were detected with the HRP labelled mouse anti-E tag antibody and a FITC-conjugated sheep anti- mouse IgG antibody (diluted 1 : 125 in PBS/2% FCS, Sigma). Cells were resuspended in 500µl PBS/2% FCS containing 10 µg/ml Propidiumjodid and measured with a FACSCalibur™ (Becton Dickinson).
(b) For analysing the expression of VEGFR-2 on the surface of cultivated HUVE cells, they were freshly prepared out of the umbilical cord. One half of cells were analysed directly by flow cytometry for surface expression of CD34 and VEGFR-2. The rest of cells were cultivated using endothelial cell Basal Medium-2 (clonetics) and analysed after different passages. Expression of VEGFR-2 was detected with scFv A7 and expression of CD34 was directly detected with a phycoerythrin labelled mouse anti-CD34 antibody (Clone No. 581) (diluted 1:10 in PBS/2% FCS, Becton Dickonson, San Jose,CA) and analyzed as in (a).
(c) VEGFR-2 expression on the cell surface of human cord blood CD 34⁺ cells was detected using the following antibodies: Two steps staining with 2µg/ml biotinylated anti-VEGFR-2 mAb (clone 260.4, Sigma) followed by staining with streptavidin-PE (Pharmingen, San Diego, CA, USA). Three steps staining with 0.5 µg/ml scFv-A7 followed by staining with the HRP labelled mouse anti-E tag antibody and than with Donkey anti-mouse PE (Jackson, West Grove, PA, USA). FACS analysis was performed by FACsort (Becton Dickinson, San Jose, CA, USA).

### BIAcore Analysis of the soluble scFv

The binding kinetics of the soluble scFv A7 was measured using BIAcore biosensor (Pharmacia Biosensor). ScFv A7 was immobilized onto a sensor chip, and VEGFR-2 was injected at concentrations ranging from 10 to 100 nM. The Sensorgrams were evaluated using the BIA Evaluation 2.0 program and the rate constants kₒₙ and k_{off} and the dissoziation constant (K_{d}) were determined.

### Results

### Selection of recombinant anti-VEGFR-2 scFv antibodies using the immune V-gene phage display library

An immune V-gene phage display library was constructed starting from the mRNA isolated from mice immunized with the extracellular domain of VEGFR-2, to which binds specifically as well as to VEGFR-1 the ligand VEGF-A. For building up the antibody repertoire the "Recombinant Phage Antibody System" from Amersham Pharmacia Biotech was applied except the modification that after first strand cDNA synthesis the V_{H} and V_{L} domains were amplified using the primers published by Clackson et al., 1991; McCafferty et al., 1996 in 5 different PCR tubes (see Material and Methods). The yield of specific amplified and assembled scFv DNA was higher compared with the assembled scFv DNA obtained after PCR with the Heavy primers and Light primer mix from Pharmacia (data not shown). All other steps were performed according to the instructions of Pharmacia. The scFv-DNA was ligated into the pCANTAB 5 E vector, the DNA transformed by electroporation into *E.coli* XL-1-Blue and a library of 2 x 10⁶ clones obtained. After three rounds of biopanning 192 clones were randomly picked and 50 clones (26%) bound to the extracellular domain of VEGFR-2 as determined by Phage ELISA. 23 clones which gave a strong signal (OD₅₉₅ > 0.5, colour development after 30min) were sequenced and out of these 23 clones 5 differ (A2, A7, B11, G3 and H1) in the amino acid sequences of the heavy and light chains (Fig.1a and Fig.1b). The distribution of the 5 selected clones was as follows: A7 was represented 17 times (74%), A2, 11B G3, 1 time each (4%) and H1 was represented 3 times (13%). As seen from the consensus sequences all CDR domains of the variable light chains show high homology. This is similar for the CDR1 and CDR2 regions of the variable heavy chains. On the other hand, the CDR3 region of the variable heavy chain shows the most variability. A consensus sequence of only 3 amino acids could be deduced.

### Biochemical characterization of the scFv-Fragments

All 5 scFv-fragments were expressed using the nonsuppressor host *E.coli* HB2151 and purified from the supernatant by anti-E tag chromatography resulting in 1.0 - 2.5 mg soluble antibody/liter culture. All 5 scFv-fragments (A2, A7, B11, G3 and H1) did recognize specifically the extracellular domain of KDR in the native state as detected in ELISA (Table 1, at the end of this specification). No crossreaction was seen with 5% skimmed milk, lysozyme, fetal calf serum, and whole cell lysates of the insect cell line High Five not expressing the extracellular domain of VEGFR-2 (data not shown). None of the clones could detect the denatured antigen in Western Blot (Table 1, at the end of this specification). All five clones recognize the cell surface expressed VEGFR-2 on recombinant PAE/VEGFR-2 cells as shown by cell surface immunofluorescence. The most sensitive clones were A7 and A2. Additionally A7 and A2 bind very efficiently to PAE/VEGFR-2 cells and HUVE cells in FACS analysis. The clones B11, G3 and H1 recognize only very weakly PAE/VEGFR-2 cells and are therefore not suitable for FACS analysis. A7 was further tested in Immunohistology and found to be suitable to detect VEGFR-2 expressing endothelial cells in the glomerulus of human kidney tissue sections. The antibodies did not crossreact with the homologous receptor VEGFR-1 as analysed by ELISA (data not shown) .

Fig.2 shows the binding of the five antibodies to native and denatured antigen in a Dot Blot analysis. All 5 antibodies recognize the extracellular VEGFR-2 most efficiently in the native state, i.e. neither treated with SDS nor dithiotreitol (Fig. 2, row A, blots 1-5). Incubation of the antigen with SDS buffer omitting dithiotreitol for 10 min at 37°C reduces the recognition to a large extent. (Fig. 2, row B, blots 1-5). Furthermore binding to the receptor is still less stronger as in row B when it is treated with the same sample buffer for 5 min at 95°C (Fig. 2, row C, blots 1-5) and no binding is seen when the antigen is treated with Laemmli-buffer containing dithiotreitol for 5 min at 95° C (Fig. 2, row D, blots 1-5). The polyclonal mouse serum obtained from one of the three immunized mice behaves similar as the scFv-fragments (Fig. 2, blots 6, column A-D) except that it seems to contain some antibodies that recognize denatured epitope(s) of the antigen (blot 6, D). Negative controls without the primary antibodies show no reaction (Fig.2, blots 7 and 8, column A-D). The results of the experiments performed with the Laemmli-buffer containing dithiotreitol was confirmed by binding tests with overlapping peptides (15 amino acids long, offset=1, comprising the sequence of the extracellular domain of VEGFR-2) synthesized on a cellulose membrane (spot synthesis). None of the scFv-fragments recognized a linear peptide (data not shown). The results from the Dot Blot analysis and spot synthesis led to the conclusion that a conformational, not linear epitope is recognized by the antibodies.

None of the 5 clones did block binding of the ligand VEGF to the receptor VEGFR-2 as assayed in a competitive binding test (Table 1, at the end of this specification). The kinetics of scFv fragment A7 which was the most sensitive clone in FACS analysis was analysed by surface plasmon resonance with a BIAcore instrument (Table 1, at the end of this specification). The rate constants kₒₙ and k_{off} were determined to 6.5 x 10⁴ M⁻¹ x s⁻¹ and 2.5 x 10⁻⁴ s⁻¹ respectively and K_{d} was calculated from the ratio of k_{off}/kₒₙ to K_{d} = 3.8 x 10⁻⁹ M.

### Immunofluorescence and Immunohistochemistry

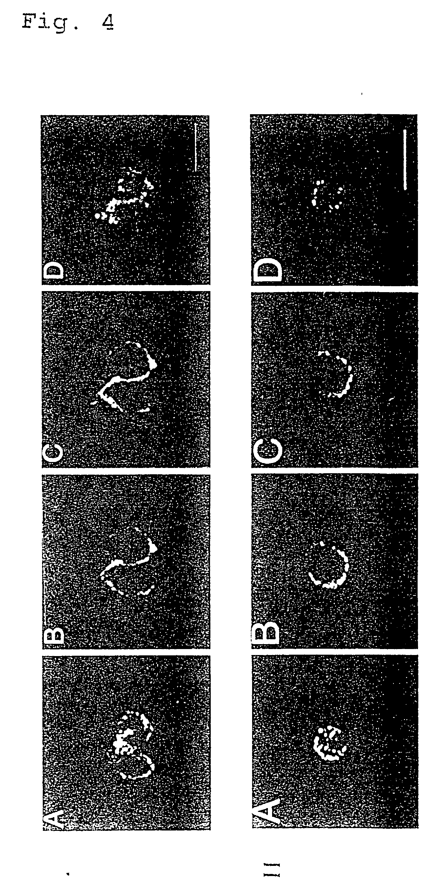

For cell surface immunofluorescence the adherent grown PAE/VEGFR-2 cells were incubated with the 5 scFv fragments and binding to the cell surface expressed VEGFR-2 analysed with a laser scanning confocal microscope (Fig. 3 and 4). In Fig. 3A binding of the clone A7 to the surface area of the cell is clearly shown and in Fig. 3B a section 2 µm underneath the surface area of the cell was stained. Binding of scFv A7 was restricted mostly to the cell membrane. In comparison to this mostly the areas around the nuclei were stained when fixed cells had been used (fixed for 30 sec at room temperature with aceton/methanol (1:1), a section 4 µm underneath the surface area is shown, Fig. 3C). In Fig. 3D and Fig. 3E controls are shown with living PAE/VEGFR-1 cells and scFv A7. No unspecific binding of scFv A7 to the cells was seen. This confirms our data that scFv A7 did not crossreact to the homologous soluble extracellular domain of VEGFR-1 in ELISA. The other 4 scFv fragments behave similarly and therefore results are not separately shown. The staining of the surface expressed VEGFR-2 on adherent cells was confirmed with scFv fragments A2 and A7 and PAE/VEGFR-2 cells in suspension. The fluorescent images representing optical sections scanned at 2 µm were collected simultaneously using the laser scanning confocal microscope and are shown in Fig. 4 I /A-D (clone A2) and Fig. 4 II/A-D (clone A7). Binding of the scFv fragments A2 and A7 to the surface of the cells was clearly evident. The VEGFR-2 seems to be unequally distributed and was observed as focal and localized patches on the cell surface. Furthermore scFv A2 and A7 recognized VEGFR-2 on the surface of VEGFR-2 cells which had been fixed with 1% paraformaldehyde for 30min on ice before staining (data not shown).

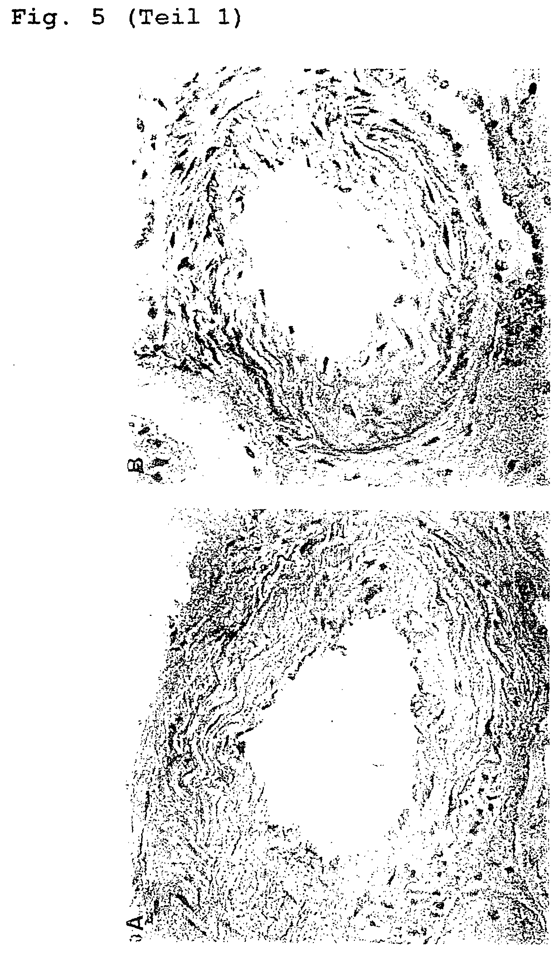

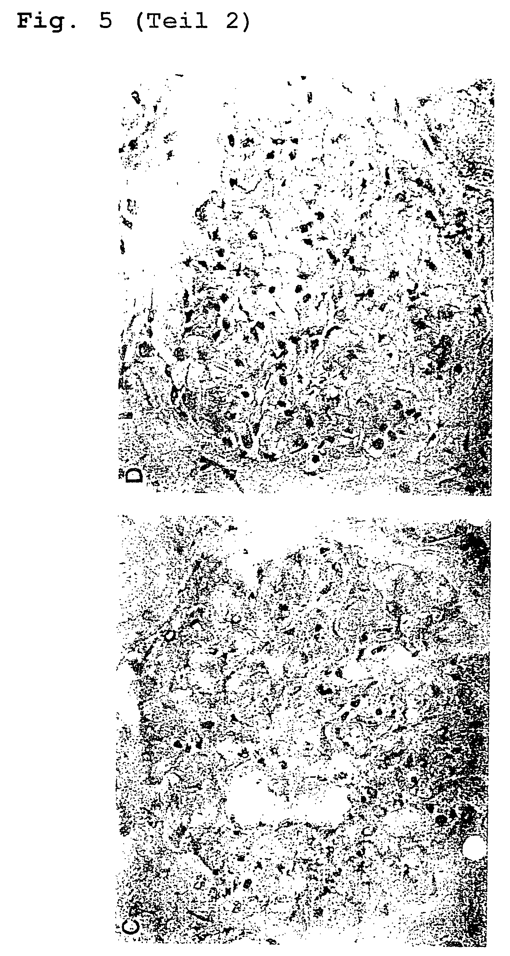

Additionally the inventors have tested the suitability of scFv A7 for immunohistochemistry on cryosections of human adult kidney tissue known to be positive for VEGFR-2 expression, Simon et al., 1998 (Fig. 5A-5D). Fig. 5A shows a red positive label for VEGFR-2 protein on a cryosection of a interlobular artery. A monolayer of endothelial cells are specifically stained inside the artery whereas the surrounding vascular smooth muscle cells are not stained. Fig. 5B shows the negative control without primary scFv A7. Fig. 5C shows a glomerulus with a red positive label for VEGFR-2 protein. The glomerular capillaries are clearly stained. Fig. 5D shows the negative control without primary scFv A7. Nuclei stained with hämatoxilin are seen in Fig. 5A-D as purple spots.

### FACS Analysis

The 5 scFv-fragments were tested for their ability to recognize the VEGFR-2 on the surface of PAE/VEGFR-2 cells. Fig. 6 shows that scFv A7 was the most efficient clone recognizing VEGFR-2. The clone A7 binds to 76.8% of the living gated cells (A), followed by A2 (B) which recognizes 54.5%. G3 (C), B11 (D), and H1 (E) bind to 8.7%, 8.2% and 3.1% of the living gated cells. Therefore scFv A7 and A2 are suitable for FACS analysis with primary cells expressing the VEGFR-2 receptor on their surface (vascular endothelial cells, progenitor cells, and hematopoietic stem cells) whereas the three other clones are not sensitive enough for FACS analysis. None of the clones show a crossreaction to PAE cells expressing VEGFR-1. Representatively this is shown for A7, A2 and G3 (Fig.6 G-I). Fig. 6 F and J shows the negative control without the primary antibodies and Fig. 6 K is the positive control with a polyclonal anti-VEGFR-1 antibody, demonstrating that the PAE/VEGFR-1 cells express the VEGFR-1 receptor.

Fig. 7A shows the amount of VEGFR-2 expressing HUVE cells in function of the number of cell passages. It is shown that the amount of VEGFR-2 positive living cells increases from 2.5% (freshly prepared cells) via 10% (1-2 passages) to 20% (3-8 passages). On the contrary the amount of CD34-positive living cells decreases depending on the number of cell passages (Fig. 7B). About 85% of freshly prepared HUVE cells are CD34 positive cells. After 4-8 passages this decreases to 5%.

Fig. 8 B and C shows that scFv A7 bind specifically to purified human cord blood CD34⁺ cells with similar sensitivity than anti-VEGFR-2 mAb clone 260.4. 0.45 % and 0.79 % of the enriched CD34⁺ cells are VEGFR-2 positive as detected with clone 260.4 and scFv A7 respectively. Fig. 8 D shows that adherent HUVE cells from the purified human cord blood expresses VEGFR-2 as clearly detected with scFv A7 (Figs. 8 D, C) and clone 260.4 (Figs. 8 D, B).

### Table 1 at the end of this specification shows the binding of the anti-VEGFR-2 scFv fragments to-VEGFR2:

^{*a*}Determined by competitive VEGF-blocking ELISA; ^{*b*}determined by direct-binding ELISA; ^{*c*}determined by Western Blotting using VEGFR-2 antigen denatured with Laemmli-buffer (PBS containing 2% SDS and 1% DTT) for 5 min at 95°C; ^{*d*}determined with living PAE/VEGFR-2 cells; ^{*e*}determined with PAE/VEGFR-2-and HUVE cells; ^{*f*} determined with human adult kidney sections; ^{*g*}determined by BIAcore analysis.

### Discussion

In the invention disclosed here the inventors have generated and analyzed several single-chain antibodies specific for the human VEGFR-2, also known as KDR receptor. They bind with high affinity to the extracellular domain, but do not block VEGF receptor interaction necessary for ligand induced proliferation and cell differentiation. The molecular characteristic, affinity and specificity of the single-chain antibodies were demonstrated by (i) the amino acid sequence of the V_{H} and V_{L} domain of the scFV fragments, (ii) dot blot analysis with soluble native and denatured antigen, (iii) cell surface immunofluorescence of VEGFR-2 overexpressing cells with laser scanning confocal microscopy, (iv) by immunostaining of endothelial cells from frozen human kidney sections, (v) binding of scFv fragments to VEGFR-2 overexpressing cells and HUVE cells and selection by flow cytometry, and (vi) for the isolation of CD34⁺/VEGFR-2⁺ stem cells from human cord blood. The results were consistent with earlier results, either using polyclonal serum or monoclonal antibodies to characterize VEGFR-2 expression, distribution and the selection of VEGFR-2 positive human primary cells (Simon et al., 1998; Menrad et al., 1997; Ziegler et al., 1999).

At first a polyclonal antibody generated in rabbit against the purified soluble receptor part was reported (Simon et al., 1998). Interesting, this antibody has a strong neutralizing activity but has been also used for immunoprezipitation and Western blotting (Morbidelli et al., 1997; Albini et al., 1996). With a similar approach, several monoclonal antibodies against the soluble receptor protein were described and characterized for different potential uses, including immunostaining techniques and Western blot studies (Simon et al., 1998; Roeckl et al., 1998; Veikkola and Alitalo, 1999). A different immunization protocol was used recently for the generation of monoclonal antibodies against the soluble receptor part (Menrad et al., 1997). Baculo-virus infected Sf-9 cells expressing the whole receptor protein in combination with a stringent and rapid screening procedure was used. This method was successful to identify two hybridomas highly specific and with high affinity for the human VEGFR-2, whereas mice immunized with isolated receptor protein alone were not successful.

VEGF ligand-receptor interaction and induction of the signalling cascade is necessary for angiogenesis, regardless of this process is induced under normal as well as pathogenic conditions. Early attemps to block this interaction leads also to the development of neutralizing antibodies, binding either to VEGF itself or to its receptor (Kim et al., 1993; Rockwell et al., 1995). An efficient receptor blocking monoclonal antibody for the mouse homolog of VEGFR-2, flk-1, was characterized in detail some years before (Rockwell et al., 1995). These studies were extended recently by the same group, establishing blocking single-chain antibodies against VEGF-R2. The approach chosen was very similar as described and selected in this study (Zhu et al., 1998). The described blocking antibodies were generated with a similar antigen but with the difference, that a fusion protein of VEGFR-2 with human alkaline phosphatase (VEGFR-2-AP) was used. The screening process was optimized to isolate and characterize 2 blocking antibodies from a library of 2.7 x 10⁸ clones (Zhu et al., 1998). From earlier studies of the inventors using the same antigen and generation of polyclonal serum in rabbits, the inventors were also expecting that some of the high affinity binders were blocking, but this observation was not made. Probably higher numbers of phages has to be screened in order to select a blocking single-chain antibody. However, from the single-chain antibodies generated and described by Zhu et al., 1998 it was not shown, that they may be used for selection of primary progenitor cells under native conditions.

In general recombinant antibodies could be successfully selected from immune-, -naive or -synthetic libraries using phage display (Hoogenboom et al., 1998). The inventors have constructed similarly than Zhu et al., 1998 an antigen-biased library because they contain antibodies with high affinity (10⁸ - 10⁹ M⁻¹, Weber-Bornhauser et al., 1998, Zhu et al., 1998) and are easier to construct than large naive- or large synthetic- libraries. A library possessing a size of 2 x 10⁶ clones was generated. The comparison of the complementary regions (CDR1-CDR3) of the heavy- and light -chains of two (the dominant nonblocker clone p2A6 and the most interesting VEGF blocker p1C11) of the 5 selected antibodies from Zhu et al., 1998 with the regions of scFv A7 showed that the CDR 3 regions of the heavy chains and light chains possessed the lowest homology. The homology was defined in percentage of identical amino acids at the sames position of the aligned sequences. Furthermore the CDR3 regions of the heavy chains of the scFv-fragments selected by Zhu et al., 1998 possessed the most variability as detected by our selected clones. From the sequence data of p2A6 and p1C11 and from the fact that p1C11 block the binding of VEGF in contrast to A7 the inventors concluded that A7 recognizes other epitopes of VEGFR-2 than p1C11 and p2A6.

For the first time the inventors could show that two of the five selected scFv-fragments (A7 and A2) of the invention recognize efficiently the membran bound VEGFR-2 on the surface of recombinant PAE/VEGFR-2 cells as shown in this invention by surface immunofluorescence and FACS analysis. Furthermore clone A7 detects human endothelial cells freshly isolated out of the umbivilical cord expressing VEGFR-2. Interestingly the expression of VEGFR-2 on the surface of the HUVE cells is dependent on the number of cell passages. Maybe the VEGF-R2 is in vitro up-regulated by the growth factors VEGF or FGF-2 which are supplements of endothelial cell Basal Medium-2. Recently this was shown for VEGF with bovine adrenal cortex endothelial cells (Shen et al., 1998). On the contrary the inventors could show that the expression of CD34 was downregulated with proliferation in continous culture and this confirms the results publisched by Delia et al., 1993. In the future the scFv fragment A7 will be very useful to study the up-regulation of VEGFR-2 expression on the surface of primary endothelial cells in more detail by FACS analysis (for example influence of hormones and growth factors).

Mesoderm inducing factors, like FGF-2, are involved in the generation of the so-called haemangioblast, which is a bipotential (so far hypothetical) precursor for the formation of angioblasts and haematopoietic stem cells (Risau and Flamme, 1995; Risau, 1997; Eichmann et al. 1997) The VEGF-R2, is an early marker of the so-called haemangioblast. After differentiation, VEGF-R2 is downregulated in differentiated haematopoietic but not in endothelial cells. The ligand for VEGF-R2, VEGF itself, acts in a paracrine fashion and it is produced by the endoderm. Threshold VEGF seems to be needed to maintain angioblast (endothelial cell precursor) differentiation as demonstrated by VEGF gene knock out experiments (Carmeliet et al., 1996; Ferrara et al., 1996). Further effidence was demonstrated by gene disruption studies which indicated, that the mouse homolog of VEGFR-2, Flk-1 is required for inititation of hematolymphopoiesis and vasculogenesis (Shalaby et al., 1997). Other studies also suggested the existence of embryonic cell positive for VEGFR-2 with hemoangiogenic potential but did not further allow identification of prenatal repopulating hematopoietic stem cells (Kabrun et al., 97; Kennedy et al., 97; Nishikawa et al., 1998). Furthermore human AC133⁺CD34⁺VEGFR-2⁺ endothelial progenitor cells have been isolated using antibodies against CD34 and it was shown that these cells have the capacity to expand and differentiate *in vitro* into mature AC133⁻VEGFR-2⁺ endothelial cells after incubation with VEGF and (FGF)-2 (Peichev et al., 2000).

Very recently it was now found, that VEGFR-2/KDR is a major functional marker for postnatal hematopoietic stem cells (HSCs, Ziegler et al, 1999). The authors purified CD34⁺ cells and separated CD34⁺VEGFR-2⁺ versus CD34⁺VEGFR-2⁻ fractions. It is shown that 0.1 to 0.5% of purified CD34⁺ cells from bone marrow, peripheral blood and cord blood were VEGFR-2⁺ and that only these cells are pluripotent stem cells whereas the CD34⁺VEGFR-2 cells are lineage-commited progenitor cells. The KDR expression was monitored with the high-affinity hybridoma clone 260.4 that binds as scFv A7 of the invention to the extracellular domain of VEGFR-2 as described by the inventors working group before (Simon et al., 1998). The most exciting fact is, that also the scFv A7 recognizes CD34⁺ cells purified by cell sorting out of human cord blood. Comparison of scFv A7 with clone 260.4 shows that scFv A7 detects the CD34⁺VEGFR-2⁺ cells with similar efficiency. Thus scFv A7 offers the possibility to purify VEGFR-2⁺ hematopoitic stem cells by cell sorting for analyzing the cellular and molecular phenotype and functional properties of these stem cells and subsets. This is very crucial for clinical applications such as stem cell transplantation and in vitro blood cell generation.

Summing up, five specific single-chain antibodies recognizing the human vascular endothelial growth factor receptor-2 (VEGFR-2/KDR) were selected from a V-gene phage display library constructed from mice immunized with the extracellular domain of VEGFR-2 (Ig-like domain 1-7). All five scFv's (A2, A7, B11, G3 and H1) bound to the purified native antigen in ELISA and Dot Blot and showed no crossreactivity to the human VEGF-receptor 1 (VEGFR-1). The selected antibodies recognize a conformation-dependent epitope of the native receptor and do not recognize denatured antigen in Western blots as well as linear overlapping peptides comprising the sequence of the human VEGFR-2. The five clones bind to the surface of endothelial cells overexpressing human VEGFR-2 c-DNA (PAE/VEGFR-2 cells) as detected by surface immunofluorescence using confocal microscopy. In addition A7 specifically detected VEGFR-2 expressing endothelial cells in the glomerulus of frozen human kidney tissue sections. Therefore A7 has potential clinical application as a marker for angiogenesis in cryosections of different human tissues. Additionally and for the first time two recombinant scFv's (A2 and A7) recognize very efficiently VEGFR-2 on PAE/VEGFR-2 cells and on human umbilical vein endothelial cells by FACS analysis. The scFv fragment A7 which was the most sensitive clone in FACS analysis recognizes human CD34⁺VEGFR-2⁺ hematopoietic immature cells within the population of enriched CD34⁺ cells that were isolated from human cord blood. The dissociation constant of A7 was determined to K_{d} = 3.8 x 10⁻⁹ M by BIAcore analysis. In conclusion, scFv fragment A7 seems to be an important tool for FACS analysis and cell sorting of vascular endothelial cells, progenitor cells and hematopoitic stem cells, which are positive for VEGFR-2 gene expression.

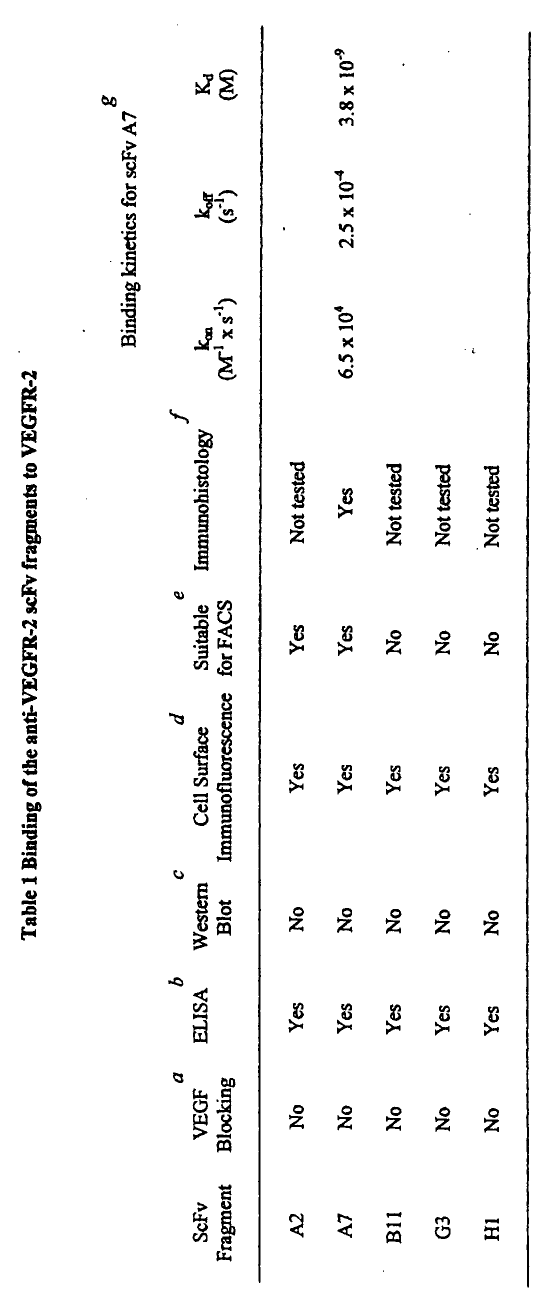

### References

Albini, A., Soldi, R., Giunciuglio, D., Giraudo, E., Benelli, R., Primo, L., Noonan, D., Salio, M., Camussi, G., Roeckl, W. and Bussolino, F. (1996) The angiogenesis induced by HIV-1 Tat protein is mediated by the Flk-1/KDR receptor on vascular endothelial cells. Nat. Med., 2 (12), 1371-1375.

Asahara, T., Murohara, T., Sullivan, A., Silver, M., van der Zee, R., Li, T., Witzenbichler, B., Schatteman, G., and Isner, J.M. (1997) Isolation of putative progenitor endothelial cells for angiogenesis. Science, 275, 964-7.

Carmeliet, P., Ferreira, V., Breier, G., Pollefeyt, S., Kieckens, L., Gertsenstein, M., Fahrig, M., Vandenhoeck, A., Harpal, K., Eberhardt, C., Declercq, C., Pawling, J., Moons, L., Collen, D., Risau, W. and Nagy, A. (1996) Abnormal blood vessel development and lethality in embryos lacking a single VEGF allele. Nature (London), 380, 435-9

Chomczynski, P., and Sacchi, N. (1987) Single-step method RNA isolation by acid guanidinium thiocyanate-phenolchloroform extraction. Anal. Biochem. 162, 156-159.

Clackson, T., Hoogenboom, H.R., Griffiths, A.D. and Winter G. (1991) Making antibody fragments using phage display libraries. Nature (London) 352, 624-628.

Delia, D., Lampugnani, M.G., Resnati, M., Dejana, E., Aiello, A., Fontanella, E.., Soglio, D., Pierotti, M.A. and Greaves M.F. (1993) CD34 expression is regulated reciprocally with adhesion molecules in vascular endothelial cells in vitro. Blood, 81(4), 1001-1008.

Eichmann, A., Corbel, C., Nataf, V., Vaigot, P., Breant, C., Le Douarin, N.M. (1997) Ligand-dependent development of the endothelial and hemopoitic lineages from embryonic mesodermal cells expressing vascular endothelial growth factor receptor 2. Proc. Natl. Acad. Sci. USA, 94 (10), 5141-6

Ferrara, N., Carvermoore, K., Chen, H., Dowd, M., Lu, L., Oshea, K., S., Powellbraxton, L., Hillan, K.J. and Moore, M.W. (1996) Heterozygous embryonic lethality induced by targeted inactivation of the VEGF gene. Nature (London) 380, 439-442.

Ferrara, N. and Davis-Smyth, T. (1997) The biology of vascular endothelial growth factor. Endocr. Rev. 18, 4-25. Hoogenboom, H.R., de Bruïne, A.P., Hufton, S.E., Hoet, R.M., Arends, J.-W. and Roovers, R.C. (1998) Antibody phage display technology and ist applications. Immunotechnology 4, 1-20.

Kabrun, N., Buhring, H.J., Choi, K., Ullrich, A., Risau, W. and Keller, G. (1997) Flk-1 expression defines a population of early embryonic hematopoietic precursors. Development 124 (10), 2039-48

Kennedy M., Firpo, M., Choi, K., Wall, C., Robertson, S., Kabrun, N. and Keller, G. (1997) A common precursor for primitive erythropoiesis and definitive haematopoiesis, Nature (London), 386, 488-93

Kim, K.J., Li, B., Winer J., Armanini, M., Gillet, N., Phillips, H.S. and Ferrara, N. (1993) Inhibition of vascular endothelial growth factor-induced angiogenesis suppresses tumor growth in vivo. Nature (London), 362, 5822-5827.

Leung, D.W., Cachianes, G., Kuang, W.-J., Goeddel, D.V. and Ferrara, N. (1989) Vascular endothelial growth factor is a secreted angiogenic mitogen. Science, 246, 1306-9.

McCafferty, J., Hoogenboom, H.R. and Chiswell., D.J. (1996) Antibody engineering. A practical laboratory manual. Academic Press. New York.

Menrad, A., Thierauch, K.-H., Martiny-Baron, G., Siemeister, G., Schirner, M. and Schneider, M.R. (1997) Novel antibodies directed against the extracellular domain of the human VEGF-receptor type II. Hybridoma, 16(5), 465-471.

Millauer, B., Wizigmann-Voss, S., Schnurch, H., Martinez, R., Moller, N.P., Risau, W. and Ullrich, A. (1993) High affinity VEGF binding and developmental expression suggest Flk-1 as a major regulator of vasculogenesis and angiogenesis. Cell, 72(6), 835-46.

Morbidelli, L., Birkenhaeger, R., Roeckl, W., Granger, H.J., Kaerst, U., Weich, H.A. and Ziche, M. (1997) Distinct capillary density and progrssion promoted by vascular endothelial growth factor-A homodimers and heterodimers. Angiogenesis, 1, 117-130.

Nishikawa, S.I., Nishikawa, S., Hirashima, M., Matsuyoshi, N. and Kodama, H. (1998) Progressive lineage analysis by cell sorting and culture identifies FLK1+VE-cadherin+ cells at a diverging point of endothelial and hemopoietic lineages. Development, 125 (9), 1747-57

Quadros, E.V., Rothenberg, S.P., and Jaffe E.A. (1989) Endothelial cells from human umbilical vein secrete functional transcobalamin II. Am. J. Physiol. 256, 296-303.

Quinn, T.P., Peters, K.G., De Vries, C., Ferrara, N. and Williams, L.T. (1993) Fetal liver kinase 1 is a receptor for vascular endothelial growth factor and is selectively expressed in vascular endothelium. Proc. Natl. Acad. Sci. USA, 90, 7533-7537

Risau, W. (1997) Mechanisms of angiogenesis. Nature (London), 386, 671-674.

Risau, W. and Flamme, I. (1995) Vasculogenesis. Annu. Rev. Cell Dev. Biol., 11, 73-91

Rockwell, P., Neufeld, G., Glassmann, A., Caron, D. and Goldstein, N. (1995) In vitro neutralization of vascular endothelial growth factor activation of Flk-1 by a monoclonal antibody. Mol. Cell. Differentation, 3, 91-109.

Roeckl, W., Hecht, D., Sztajer, H., Waltenberger, J., Yayon, A. and Weich, H.A. (1998) Differential binding characteristics and cellular inhibition by soluble VEGF receptors 1 and 2. Exp. Cell. Res. 241, 161-170.

Shalaby, F., Ho, J., Stanford, W.L., Fischer, K.D., Schuh, A.C., Schwartz, L., Bernstein, A. and Rossant, J. (1997) A requirement for Flk1 in primitive and definitive hematopoiesis and vasculogenesis. Cell, 89(6), 981-90

Shen, B.Q., Lee,D.Y., Gerber, H.-P., Keyt, B.A., Ferrara, N. and Zioncheck, T.F. (1998) Homologous up-regulation of KDR/Flk-1 receptor expression by vascular endothelial growth factor in vitro. J. Biol. Chem., 273(45) 29979-29985.

Simon, M., Röckl, W., Hornig, C., Gröne, E.F., Theis, H., Weich, H.A., Fuchs, E., Yayon, A. and Gröne, H.-J. (1998) Receptors of vascular endothelial growth factor/vascular permeability factor (VEGF/VPF) in fetal and adult human kidney: localisation and [¹²⁵I] VEGF binding sites. Journal of the American Society of Nephrology, 1032-1044.

Veikkola, T. and Alitalo, K. (1999) VEGFs, receptors and angiogenesis. Cancer Biology, 9, 211-220.

Waltenberger, J., Claesson-Welsh, L., Siegbahn, A., Shibuya, M. and Heldin, C.H. (1994) Different signal transduction properties of KDR and Flt1, two receptors for vascular endothelial growth factor. J Biol Chem 269 (43), 26988-95.

Weber-Bornhauser, S., Eggenberger, J., Jelesarov, I., Bernard, A., Berger C. and Bosshard, H.R. (1998) Thermodynamics and kinetics of the reaction of a single-chain antibody fragment (scFv) with the leucine zipper domain of transcription factor GCN4. Biochemistry, 37, 13011-13020.

Wilting, J., Christ, B. and Weich, A. W. (1992) The effects of growth factors on the day 13 chorioallantoic membran (CAM): a study of VEGF₁₆₅ and PDGF-BB. Anat. Embryol., 186, 251-257.

Zhu, Z., Rockwell, P., Lu, D., Kotanides, H., Pytowski, B., Hicklin, D.J., Bohlen, P. and Witte, L. (1998) Inhibition of vascular endothelial growth factor-induced receptor activation with anti-kinase insert domain-containing receptor single-chain antibodieas from a phage display library. Cancer Res. 58, 3209-3214.

Peichev, M.., Naiyer, A.J., Pereira, D., Zhu, Z., Lane, W.J., Williams, M., Oz, M.C., Hicklin, D.J., Witte, L., Moore, M.A.S. and Shahin, R. (2000) Expression of VEGFR-2 and AC133 by circulating human CD34⁺ cells identifies a population of functional endothelial precursors. Blood. 95 (3), 952-958.

Ziegler, B.L., Valtieri, M., Almeida Porada, G., De Maria, R., Müller, R., Masella, B., Gabbianelli, M., Casella, I., Pelosi, E., Bock, T., Zanjani, E.D. and Peschle, C. (1999) KDR Receptor: A key marker defining hematopoietic stem cells. Science, 285, 1553-1558.

## Claims

1. Single-chain antibody recognizing the human vascular endothelial growth factor receptor-2 selected from the group consisting of:
(a) a polypeptide having the following general structure:
(V_{H} domain)-Linker-(V₁ domain),
wherein said V_{H} domain comprises the following amino acid sequence: 
Gln Val Gln Leu Gln Gln Ser Gly Pro Glu Leu Val Lys Pro Gly
Ala Ser Val Lys Ile Ser Cys Lys Thr Ser Gly Tyr Thr Phe Thr
Glu Tyr Thr Met His Trp Val Lys Gln Ser His Gly Lys Ser Leu
Glu Trp Ile Gly Gly Ile Asn Pro Asn Thr Gly Gly Thr Ser Tyr
Asn Gln Lys Phe Lys Gly Lys Ala Thr Leu Thr Val Asp Lys Ser
Ser Ser Thr Ala Tyr Met Glu Leu Arg Ser Leu Thr Ser Glu Asp
Ser Ala Val Tyr Tyr Cys Ala Arg Gly His Tyr Ser Ser Ser Leu
Asp Tyr Trp Gly Gln- (scFv A2)
 or 
Gln Val Lys Leu Gln Glu Ser Gly Pro Glu Leu Val Lys Pro Gly
Thr Ser Val Lys Ile Ser Cys Lys Thr Ser Gly Tyr Thr Phe Thr
Glu Tyr Thr Met His Trp Val Lys Gln Ser His Gly Lys Ser Leu
Glu Trp Ile Gly Gly Ile Asn Pro Asn Ser Gly Gly Thr Ser Tyr
Asn Gln Lys Phe Lys Gly Lys Ala Thr Leu Thr Val Asp Lys Ser
Ser Ser Thr Ala Tyr Met Glu Leu Arg Ser Leu Thr Ser Glu Asp
Ser Ala Val Tyr Tyr Cys Ala Arg Gly Leu Trp Gly Gly Met Asp
Tyr Trp Gly Gln- (scFv A7),
 
and wherein said V_{L} domain comprises the following amino acid
sequence:
 
-Thr Ile Met Ser Ala Ser Pro Gly Glu Lys Val Thr Met Thr Cys
Ser Ala Ser Ser Ser Val Ser Tyr Met His Trp Tyr Gln Gln Lys
Ser Gly Thr Ser Pro Lys Arg Trp Ile Tyr Asp Thr Ser Lys Leu
Ala Ser Gly Val Pro Ala Arg Phe Ser Gly Ser Gly Ser Gly Thr
 
Ser Tyr Ser Leu Thr Ile Ser Ser Met Glu Ala Glu Asp Ala Ala
Thr Tyr Tyr Cys Gln Gln Trp Thr Ser Asn Pro Phe Thr Phe Gly
Ser Gly Thr Lys Leu Glu Ile Lys Arg (scFv A2)
 or 
-Ala Ile Met Ser Ala Ser Pro Gly Glu Lys Val Thr Leu Thr Cys
Ser Ala Ser Ser Ser Val Ser Tyr Met His Trp Tyr Gln Gln Lys
Ser Ser Thr Ser Pro Lys Leu Trp Ile Tyr Asp Thr Ser Arg Leu
Ala Ser Gly Val Pro Gly Arg Phe Ser Gly Ser Gly Ser Gly Thr
Ser Tyr Ser Leu Thr Ile Ser Ser Met Glu Ala Glu Asp Ala Ala
Thr Tyr Tyr Cys Gln Gln Trp Asn Thr Tyr Pro Tyr Thr Phe Gly
Gly Gly Thr Lys Leu Glu Leu Lys Arg (scFv A7);
(b) a polypeptide which is an allelic variant of a polypeptide defined in (a) and has the same antibody activity;
(c) a mutant of a polypeptide defined in (a) or (b) in which one or more amino acid residues are deleted and/or replaced and/or inserted and/or one or more partial amino acid sequences are inverted without interfering with the antibody activity or which enhance the antibody activity;
(d) a C-terminal or N-terminal fusion protein comprising a polypeptide defined in (a) to (c) in which the additively connected amino acid residues do not interfere with the antibody activity or may be easily eliminated.

2. Single-chain antibody according to claim 1, wherein said linker is a flexible peptide comprising 2 to 28 amino acid residues.

3. Single-chain antibody according to claim 2, wherein said linker ist (Gly₄Ser)₃ or the Yol-peptide linker.

4. Single-chain antibody according to any of the preceeding claims, wherein said single-chain antibody is labeled without interfering with the antibody activity.

5. Single-chain antibody according to claim 4, wherein said label is selected from the group consisting of one or more radioactive atoms or groups, coloured or fluorescent groups, haptens, enzymes, magnetic particles, groups for immobilization to solid phases and groups for direct or indirect reactions.

6. Single-chain antibody according to claim 5, wherein said groups for indirect reactions are based on or are suitable for enzyme-conjugated secondary antibodies, the biotin/avidin(or streptavidin) system or the colloidal gold system.

7. DNA-Sequence comprising a DNA sequence selected from the group consisting of:
(a) a DNA sequence coding for a single-chain antibody according to any of claims 1 to 3, or its complementary strand;
(b) a DNA sequence which hybridizes under stringent conditions to the polypeptide coding regions of a DNA sequence defined in (a), or fragments thereof;
(c) a DNA sequence which, but for the degeneracy of the genetic code, would hybridize to a DNA sequence defined in (a) and (b);
(d) allelic Variations of a DNA sequence defined in (a) and resulting in isofunctional expression products;
(e) mutants of a DNA sequence defined in (a) to (d) in which one or more nucleotide residues are deleted and/or replaced and/or inserted and/or one or more partial nucleotide sequences are inverted and resulting in isofunctional expression products.

8. Recombinant expression vector comprising a DNA sequence according to claim 7.

9. Procaryotic or eucaryotic cell transformed or transfected with a DNA sequence according to claim 7 and/or a recombinant expression vector according to claim 8.

10. Cell according to claim 9, wherein the cell is derived from an E. coli strain.

11. Process for the preparation of a single-chain antibody according to any of claims 1 to 3, comprising the steps of cultivating cells according to claim 9 or 10 in a suitable culture medium and isolating said single-chain antibody from said cells and/or said medium.

12. Expression product or partial expression product of a DNA sequence according to claim 7 or of an expression vector according to claim 8.

13. Use of a single-chain antibody according to any of claims 1 to 6 as an immunohistochemical marker for angiogenesis or vascularization in a human tissue.

14. Use according to claim 13, wherein said tissue is from a cryosection.

15. Use according to claim 13 or 14, wherein said tissue is a growing carcinoma.

16. Use of a single-chain antibody according to any of claims 1 to 6 for FACS analysis and cell sorting of cells expressing human vascular endothelial growth factor receptor-2 on their surfaces.

17. Use according to claim 16, wherein the cells expressing human vascular endothelial growth factor receptor-2 are selected from the group consisting of human vascular endothelial cells and their progenitor cells, human megacaryocytes and their progenitor cells, human hematopoitic stem cells from bone marrow, umbical cord blood or mobilized peripheral blood.

18. Use according to claims 16 or 17, wherein said cell sorting is performed by using flow cytometry or by antibody or magnetic particle mediated selection without blocking growth factor induced cell proliferation and differentiation during selection and propagation.

19. Use of a single-chain antibody according to any of claims 1 to 6 for vascular and stem cell targeting a drug or toxin, a radionuclide, a gene or a viral coat protein conjugated thereto.

20. Kit comprising at least one single-chain antibody according to any of claims 1 to 6 for the uses defined in any of claims 13 to 19.
